(19) Europäisches Patentamt
European Patent Office
Office européen des brevets

(11) Veröffentlichungsnummer: **0 028 355**
**B1**

(12) # EUROPÄISCHE PATENTSCHRIFT

(45) Veröffentlichungstag der Patentschrift: **09.05.84**

(51) Int. Cl.³: **C 07 D 261/08**, C 07 D 261/18, C 07 D 413/12, A 01 N 43/74

(21) Anmeldenummer: **80106427.0**

(22) Anmeldetag: **22.10.80**

(54) **1,2-Oxazolylalkylcarbamate, ihre Herstellung, ihre Verwendung als Herbizide und herbizide**

(30) Priorität: **31.10.79 DE 2943965**

(43) Veröffentlichungstag der Anmeldung: **13.05.81 Patentblatt 81/19**

(45) Bekanntmachung des Hinweises auf die Patenterteilung: **09.05.84 Patentblatt 84/19**

(84) Benannte Vertragsstaaten: **AT BE CH DE FR GB IT LI NL SE**

(56) Entgegenhaltungen:
**DE - A - 1 812 761**
**DE - A - 2 633 790**
**DE - A - 2 823 384**

**Die Akte enthält technische Angaben, die nach dem Eingang der Anmeldung eingereicht wurden und die nicht in dieser Patentschrift enthalten sind.**

(73) Patentinhaber: **BASF Aktiengesellschaft, Carl-Bosch-Strasse 38, D-6700 Ludwigshafen (DE)**

(72) Erfinder: **Theobald, Hans, Dr., Parkstrasse 2, D-6703 Limburgerhof (DE)**
Erfinder: **Wuerzer, Bruno, Dr., Ruedigerstrasse 13, D-6701 Otterstadt (DE)**
Erfinder: **Klehs, Karl, Dr., Sudetenstrasse 22, D-6840 Lampertheim (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



ACTORUM AG

**Beschreibung**

Die vorliegende Erfindung betrifft 1,2-Oxazolylalkylcarbamate, ein Verfahren zu ihrer Herstellung, Herbizide, die diese Verbindungen als Wirkstoffe enthalten, sowie ein Verfahren zur Bekämpfung unerwünschten Pflanzenwuchses mit diesen Verbindungen.

Es ist bekannt, dass sich Carbamate zur Bekämpfung unerwünschten Pflanzenwuchses eignen. So bekämpft N-Phenylcarbaminsäureisopropylester Gräser (DE-PS 833 274), wobei die Wirkung vornehmlich über die Wurzeln erfolgt. Um das Mittel mit den Wurzeln in Kontakt zu bringen, ist darum genügend Bodenfeuchtigkeit erforderlich, so dass die Wirksamkeit des Herbizids von den jeweiligen Niederschlagsverhältnissen abhängig ist.

Eine Verbesserung der herbiziden Wirkung bei der bevorzugten Vorauflaufanwendung bringt der N-3--Chlorphenyl-carbaminsäure-isopropylester (US-A-2 695 225). Die Verwendung von N-3,4-Dichlorphenyl-carbaminsäure-methylester zur Bekämpfung von unerwünschten Pflanzen bei Vor- und Nachauflaufanwendung wurde ebenfalls beschrieben (US-A-3 116 995). Der Wirkstoff ist zur Unkrautbekämpfung in grossamigen Leguminosen und in Reis geeignet, wobei die Applikation im Vorauflaufverfahren oder im frühen Nachauflauf auf sehr kleine Pflanzen erfolgen sollte.

Weiterhin sind 1,2-Oxazolylmethylthiolcarbamate mit herbizider Wirksamkeit bekannt (DE-A-2 633 790). Im Vergleich zu vorher bekannten Thiolcarbamaten haben diese Verbindungen ein anderes und breiteres Wirkungsspektrum. Bei ihrer Anwendung erübrigt sich wegen des geringen Dampfdrucks eine Einarbeitung vor der Saat.

Ausserdem sind aus der DE-A-2 823 384 herbizide 1,2-Oxazolyl-methyl-N-aryl-thiolcarbamate bekannt. Die Wirkung dieser Thiolcarbamate bei niedrigen Aufwandmengen ist jedoch schwach.

Es wurde gefunden, dass 1,2-Oxazolylalkylcarbamate der Formel I

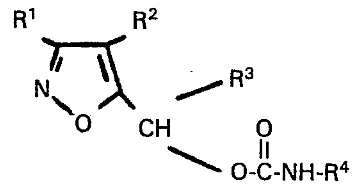

I

in der

$R^1$ ein Alkoxyalkylrest mit bis zu 3 Kohlenstoffatomen oder ein Alkoxycarbonylrest mit bis zu 5 Kohlenstoffatomen,

$R^2$ Wasserstoff oder ein Alkylrest mit bis zu 3 Kohlenstoffatomen, der durch Halogen substituiert sein kann,

$R^3$ Wasserstoff oder ein Alkylrest mit bis zu 3 Kohlenstoffatomen und

$R^4$ ein Phenylrest, der durch Halogen, Nitro, Cyano oder Alkyl-, Alkoxy-. Halogenalkyl-, Halogenalkoxy-, Alkanoyl- oder Alkoxycarbonylgruppen mit bis zu 5 Kohlenstoffatomen oder Cycloalkylgruppen mit bis zu 6 Kohlenstoffatomen substituiert sein kann,

sind, sowohl bei Vor- als auch bei Nachauflaufanwendung herbizid wirksam und gleichzeitig kulturpflanzenverträglich sind. Sie sind den bekannten herbiziden (Thiol)Carbamaten in ihrer Wirkung gegen breitblättrige unerwünschte Pflanzen überlegen.

In der Formel I der 1,2-Oxazolylalkylcarbamate, bedeuten $R^1$ einen unverzweigten oder verzweigten Alkoxyalkylrest mit bis zu 3 Kohlenstoffatomen, wie Methoxymethyl, Methoxyethyl, oder einen Alkoxycarbonylrest mit bis zu 5 Kohlenstoffatomen, insbesondere mit bis zu 4 Kohlenstoffatomen, wie Methoxycarbonyl, Ethoxycarbonyl, n-Propoxycarbonyl, i-Propoxycarbonyl sowie die 4 isomeren Butoxycarbonylreste,

$R^2$ ausser Wasserstoff einen gegebenenfalls durch Halogen substituierten Alkylrest mit bis zu 3 Kohlenstoffatomen, wie Methyl, Chlormethyl, Ethyl, 2-Chlorethyl, n-Propyl, i-Propyl,

$R^3$ ausser Wasserstoff einen Alkylrest mit bis zu 3 Kohlenstoffatomen, wie Methyl, Ethyl oder die Propylreste, und

$R^4$ einen Phenylrest, der durch Halogen, Nitro, Cyano, unverzweigte oder verzweigte Alkyl-, Alkoxy-, Halogenalkyl-, Halogenalkoxy-, Alkanoyl od. Alkoxycarbonylgruppen mit jeweils bis zu 5 Kohlenstoffatomen, insbesondere bis zu 3 Kohlenstoffatomen, oder Cycloalkylreste mit 3 bis 6 Kohlenstoffatomen einfach oder mehrfach substituiert sein kann, wie Phenyl, 3-Chlorphenyl, 3,4-Dichlorphenyl, 3-Fluor--4-chlorphenyl,4-Fluorphenyl, 3-Chlor-4-fluorphenyl, 4-Chlorphenyl, 4-Bromphenyl, 2,5-Difluorphenyl, 3-Nitro-4-methylphenyl, 3-Chlor-4-cyanophenyl, 3-Chlor-4-methylphenyl, 3-Methylphenyl, 2-Isopropylphenyl, 4-Isopropylphenyl, 4-tert.-Butylphenyl, 3,4-Dimethylphenyl, 2-Ethylphenyl, 2,6-Diethylphenyl, 3-Trifluormethylphenyl, 3-Trifluormethyl-4-chlorphenyl, 2-Methyl-3-chlorphenyl, 2--Methoxy-4-chlorphenyl, 2,4-Dimethoxy-5-chlorphenyl, 4-Methoxy-3-chlorphenyl, 4-Difluormethoxyphenyl, 3-Methoxy-4-methylphenyl, 4-Cyclopropylphenyl, 4-Cyclohexylphenyl, 4-Acetylphenyl, 4-Propionylphenyl, 4-Methoxycarbonylphenyl, 4-Ethoxycarbonylphenyl, 3-Ethoxycarbonylphenyl.

Man erhält die 1,2-Oxazolylalkylcarbamate der Formel I durch Umsetzung von 5-Hydroxymethyl--1,2-oxazolen der Formel II

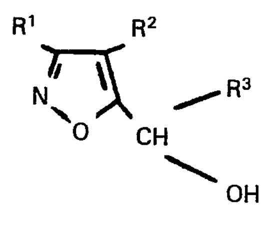

II

in der $R^1$, $R^2$ und $R^3$ die obengenannten Bedeutungen haben, mit Isocyanaten der Formel III

$$OCN\text{-}R^4 \qquad III$$

in der $R^4$ die obengenannten Bedeutungen hat, gegebenenfalls in Anwesenheit eines Verdünnungsmittels.

Die Umsetzung wird bei einer Temperatur zwischen —50 und +150°C, vorzugsweise zwischen

+20 und +60°C, durchgeführt. Es kann zweckmässig sein, in Anwesenheit eines Verdünnungsmittels und gegebenenfalls auch in Anwesenheit eines Katalysators zu arbeiten.

Geeignete Verdünnungsmittel sind aromatische und aliphatische, gegebenenfalls chlorierte Kohlenwasserstoffe, wie Benzol, Toluol, Kresole, Halogenbenzole, Methylenchlorid, Chloroform, Tetrachlorkohlenstoff, Ether, wie Diethylether, Tetrahydrofuran, Dioxan, Ketone, wie Aceton, Diethylketon, Methylethylketon, Diisopropylketon, sowie Acetonitril, Dimethylformamid und Dimethylsulfoxid. Als Katalysatoren kommen tertiäre Amine, wie Triethylamin, in Betracht.

Die als Ausgangsstoffe eingesetzten 5-Hydroxymethyl-1,2-oxazole der Formel II sind teilweise bekannt und können in Anlehnung an bekannte Verfahren hergestellt werden [Tetrahedron Letters *4*, 327-330 (1967)]. Man erhält so beispielsweise folgende 5-Hydroxymethyl-1,2-oxazole der Formel II

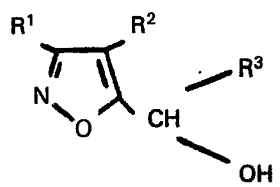

| $R^1$ | $R^2$ | $R^3$ | $n_D^{25}$/Kp |
|---|---|---|---|
| $CH_3OCH_2$- | H | H | 1,4778 |
| $CH_3OCH_2$- | H | $CH_3$ | 118-121°C/1,1 mbar |
| $C_2H_5OOC$- | H | $CH_3$ | 1,4740 |

Die Isocyanate der Formel $R^4$-NCO sind bekannt bzw. können nach bekannten Methoden leicht hergestellt werden (Houben-Weyl, Methoden der organischen Chemie, Band 8, S. 119-128, Georg Thieme-Verlag, Stuttgart, 1952).

Die erfindungsgemässen 1,2-Oxazolylalkylcarbamate der Formel I können analog folgendem Beispiel hergestellt werden:

*Beispiel 1*

13,5 Gewichtsteile 3-Isobutyl-5-(1'-hydroxyethyl)-1,2-oxazol werden in 150 Volumenteilen Toluol gelöst und nach Zugabe von 0,1 Gewichtsteilen Triethylamin mit 9,5 Gewichtsteilen Phenylisocyanat tropfenweise versetzt. Danach wird 1 Stunde bei Raumtemperatur und 4 Stunden bei 50°C gerührt. Nach dem Abkühlen wird der Niederschlag abgesaugt und mit Petroläther gewaschen. Man erählt 14 Gewichtsteile 1-(3-Isobutyl-1,2-oxazolyl-5)-ethyl-N-phenylcarbamat als kristalline Substanz vom Schmelzpunkt 105°C.

$C_{16}H_{20}N_2O_3$ (288)

Ber.: C 66,7 H 7,0 N 9,7

Gef.: C 66,7 H 6,5 N 10,3

Entsprechend können beispielsweise folgende Verbindungen der Formel

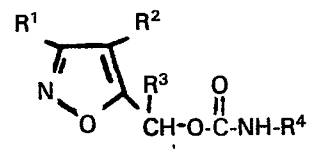

erhalten werden:

| Nr. | $R^1$ | $R^2$ | $R^3$ | $R^4$ | Fp (°C) / δ-Werte (MHz; LM) |
|---|---|---|---|---|---|
| 1 | $CH_3OCH_2$- | H | H | 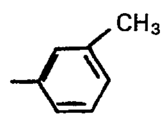 | [80; $D_6$DMSO] 2,3 (3H); 4,5 (2H), 5,3 (2H), 6,1 (1H), 6,7-7,4 (4H) |
| 2 | $CH_3OCH_2$- | H | H | 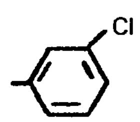 | 84-85 |
| 3 | $CH_3OCH_2$- | H | $CH_3$ | 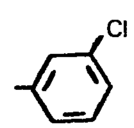 | [100; $D_6$DMSO] 1,7 (3H), 3,3 (3H), 4,45 (2H), 6,0 (1H), 6,5 (1H), 6,9-7,6 (4H) |
| 4 | $CH_3OCH_2$- | H | $CH_3$ | 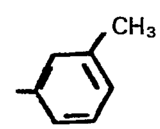 | [100; $D_6$DMSO] 1,45 (3H), 2,3 (3H), 3,3 (3H), 4,42 (2H), 5,96 (1H), 6,5 (1H), 6,7-7,3 (4H) |
| 5 | $H_5C_2OOC$- | H | $CH_3$ | 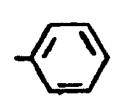 | 59-61 |

| Nr. | $R^1$ | $R^2$ | $R^3$ | $R^4$ | Fp (°C) / δ-Werte (MHz; LM) |
|---|---|---|---|---|---|
| 6 | $H_5C_2OOC$- | H | $CH_3$ | 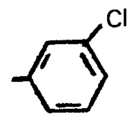 | 103-104 |
| 7 | $H_5C_2OOC$- | H | $CH_3$ | 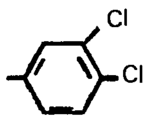 | 143-144 |
| 8 | $H_5C_2OOC$- | H | $CH_3$ | 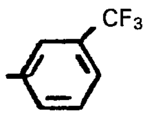 | 104-106 |
| 9 | $CH_3OCH_2$- | H | $CH_3$ | 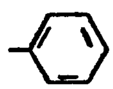 | [60; $D_6DMSO$] 1,6 (3H), 3,3 (3H), 4,4 (2H), 5,9 (1H), 6,2 (1H), 6,9-7 (5H), 7,7 (1H) |
| 10 | $CH_3OCH_2$- | H | $CH_3$ | 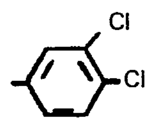 | [270;$CDCl_3$+ $D_6DMSO$] 1,74 (3H), 3,25 (3H), 4,45 (2H), 6,05 (1H), 6,32 (1H), 7,18 (2H), 7,75 (1H), 8,44 (1H) |
| 11 | $CH_3OCH_2$- | H | $CH_3$ | 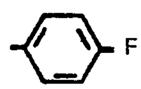 | [60; $D_6DMSO$] 1,5 (3H), 3,15 (3H), 4,35 (2H), 5,9 (1H), 6,45 (1H), 6,8-7,5 (4H), 9,8 (1H) |
| 12 | $CH_3OCH_2$- | H | 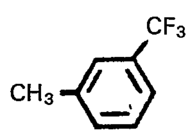 | | [60; $D_6DMSO$] 1,65 (3H), 3,2 (3H), 4,36 (2H), 5,9 (1H), 6,47 (1H), 7,2-7,85 (4H) |

Die erfindungsgemässen Wirkstoffe können beispielsweise in Form von direkt versprühbaren Lösungen, Pulvern, Suspensionen, auch hochprozentigen wässrigen, öligen oder sonstigen Suspensionen oder Dispersionen, Emulsionen, Öldispersionen, Pasten, Stäubemitteln, Streumitteln oder Granulaten durch Versprühen, Vernebeln, Verstäuben, Verstreuen oder Giessen angewendet werden. Die Anwendungsformen richten sich ganz nach den Verwendungszwecken; sie sollten in jedem Fall möglichst die feinste Verteilung der erfindungsgemässen Wirkstoffe gewährleisten.

Zur Herstellung von direkt versprühbaren Lösungen, Emulsionen, Pasten oder Öldispersionen kommen Mineralölfraktionen von mittlerem bis hohem Siedepunkt, wie Kerosin oder Diselöl, ferner Kohlenteeröle sowie Öle pflanzlichen oder tierischen Ursprungs, aliphatische, cyclische und aromatische Kohlenwasserstoffe, z.B. Benzol, Toluol, Xylol, Paraffin, Tetrahydronaphthalin, alkylierte Naphthaline oder deren Derivate, z.B. Methanol, Ethanol, Propanol, Butanol, Chloroform, Tetrachlorkohlenstoff, Cyclohexanol, Cyclohexanon, Chlorbenzol, Isophoron, stark polare Lösungsmittel, wie z.B. Dimethylformamid, Dimethylsulfoxid, N-Methylpyrrolidon, Wasser, in Betracht.

Wässrige Anwendungsformen können aus Emulsionskonzentraten, Pasten oder netzbaren Pulvern (Spritzpulvern, Öldispersionen) durch Zusatz von Wasser bereitet werden. Zur Herstellung von Emulsionen, Pasten oder Öldispersionen können die Substanzen als solche oder in einem Öl oder Lösungsmittel gelöst, mittels Netz-, Haft-, Dispergier- oder Emulgiermittel in Wasser homogenisiert werden. Es können aber auch aus wirksamer Substanz Netz-, Haft-, Dispergier- oder Emulgiermittel und eventuell Lösungsmittel oder Öl bestehende Konzentrate hergestellt werden, die zur Verdünnung mit Wasser geeignet sind.

Als oberflächenaktive Stoffe kommen Alkali-, Erdalkali-, Ammoniumsalze von Ligninsulfonsäure, Naphthalinsulfonsäure, Phenolsulfonsäure, Alkylarylsulfonate, Alkylsulfate, Alkylsulfonate, Alkali- und Erdalkalisalze der Dibutylnaphthalinsulfonsäure, Laurylethersulfat, Fettalkoholsulfate, fettsaure Alkali- und Erdalkalisalze, Salze sulfatierter Hexade-

canole, Heptadecanole, Octadecanole, Salze von sulfatiertem Fettalkoholglykolether, Kondensationsprodukte von sulfoniertem Naphthalin und Naphthalinderivaten mit Formaldehyd, Kondensationsprodukte des Naphthalins bzw. der Naphthalinsulfonsäuren mit Phenol und Formaldehyd, Polyoxyethylenoctylphenoläther, ethoxyliertes Isooctylphenol, Octylphenol, Nonylphenol, Alkylphenolpolyglykolether, Tributylphenylpolyglykolether, Alkylarylpolyetheralkohole, Isotridecylalkohol, Fettalkoholethylenoxid-Kondensate, ethoxyliertes Rizinusöl, Polyoxyethylenalkylether, ethoxyliertes Polyoxypropylen, Laurylalkoholpolyglykoletheracetal, Sorbitester, Lignin, Sulfitablaugen und Methylcellulose in Betracht.

Pulver-, Streu- und Stäubemittel können durch Mischen oder gemeinsames Vermahlen der wirksamen Substanzen mit einem festen Trägerstoff hergestellt werden.

Granulate, z.B. Umhüllungs-, Imprägnierungs- und Homogengranulate, können durch Bindung der Wirkstoffe an festen Trägerstoffen hergestellt werden. Feste Trägerstoffe sind Mineralerden wie Silicagel, Kieselsäuren, Kieselgele, Silikate, Talkum, Kaolin, Attaclay, Kalkstein, Kalk, Kreide, Bolus, Löss, Ton, Dolomit, Diatomeenerde, Calcium- und Magnesiumsulfat, Magnesiumoxid, gemahlene Kunststoffe, Düngemittel, wie z.B. Ammoniumsulfat, Ammoniumphosphat, Ammoniumnitrat, Harnstoffe und pflanzliche Produkte, wie Getreidemehl, Baumrinden-, Holz- und Nussschalenmehl, Cellulosepulver und andere feste Trägerstoffe.

Die Formulierungen enthalten zwischen 0,1 und 95 Gewichtsprozent, vorzugsweise zwischen 0,5 und 90 Gewichtsprozent, Wirkstoff.

Beispiele für Formulierungen sind:

I. 20 Gewichtsteile der Verbindung Nr. 1 werden in einer Mischung gelöst, die aus 25 Gewichtsteilen Cyclohexanol, 65 Gewichtsteilen einer Mineralölfraktion vom Siedepunkt 210 bis 280°C und 10 Gewichtsteilen des Anlagerungsproduktes von 40 Mol Ethylenoxid an 1 Mol Ricinusöl besteht. Durch Eingiessen und feines Verteilen der Lösung in 100 000 Gewichtsteilen Wasser erhält man eine wässrige Dispersion, die 0,02 Gewichtsprozent des Wirkstoffs enthält.

II. 20 Gewichtsteile der Verbindung Nr. 2 werden mit 3 Gewichtsteilen des Natriumsalzes der Diisobutylnaphthalin-α-sulfonsäure 17 Gewichtsteilen des Natriumsalzes einer Ligninsulfonsäure aus einer Sulfit-Ablauge und 60 Gewichtsteilen pulverförmigem Kieselsäuregel gut vermischt und in einer Hammermühle vermahlen. Durch feines Verteilen der Mischung in 20 000 Gewichtsteilen Wasser erhält man eine Spritzbrühe, die 0,1 Gewichtsprozent des Wirkstoffs enthält.

III. 3 Gewichtsteile der Verbindung Nr. 7 werden mit 97 Gewichtsteilen feinteiligem Kaolin vermischt. Man erhält auf diese Weise ein Stäubemittel, das 3 Gewichtsprozent des Wirkstoffs enthält.

Die Applikation der Wirkstoffe bzw. der Mittel kann im Vorauflaufverfahren oder bei Nachauflaufanwendung erfolgen. Dabei können die Mittel auf den Standort aufgebracht werden, bevor die unerwünschten Pflanzen aus den Samen gekeimt oder aus vegetativen Pflanzenteilen ausgetrieben haben oder sie werden auf die Blätter der unerwünschten Pflanzen und der Kulturpflanzen appliziert. Eine weitere Ausbringungstechnik besteht darin, dass die Wirkstoffe mit Hilfe der Spritzgeräte so gespritzt werden, dass die Blätter der empfindlichen Kulturpflanzen nach Möglichkeit nicht getroffen werden, während die Wirkstoffe auf die darunterliegende Bodenfläche oder dort wachsende unerwünschte Pflanzen gelangen (post-directed, lay-by). Die Aufwandmengen betragen je nach Jahreszeit und Wachstumsstadium 0,1 bis 15 kg/ha und mehr, wobei sich die höheren Dosen besonders zur totalen Bekämpfung und Vegetationen eignen.

Der Einfluss verschiedener Vertreter der erfindungsgemässen 1,2-Oxazolylalkylcarbamate auf das Wachstum von unerwünschten Pflanzen im Vergleich zu bekannten Wirkstoffen wird durch Gewächshausversuche gezeigt:

Als Kulturgefässe dienten Plastikblumentöpfe mit 300 $cm^3$ Inhalt und lehmigem Sand mit etwa 1,5% Humus als Substrat. Die Samen der Testpflanzen entsprechend Tabelle 1 wurden nach Arten getrennt flach eingesät. Unmittelbar danach wurden bei Vorauflaufanwendung die Wirkstoffe auf die Erdoberfläche aufgebracht. Sie wurden hierzu in Wasser als Verteilungsmittel suspendiert oder emulgiert und mittels fein verteilender Düsen gespritzt. Nach dem Aufbringen der Mittel wurden die Gefässe leicht beregnet, um Keimung und Wachstum in Gang zu bringen und um gleichzeitig die chemischen Mittel zu aktivieren. Danach wurden die Gefässe mit durchsichtigen Plastikhauben abgedeckt, bis die Pflanzen angewachsen waren. Diese Abdeckung bewirkt ein gleichmässiges Keimen der Testpflanzen, sofern dies nicht durch die Chemikalien beeinträchtigt wird.

Zum Zwecke der Nachauflaufbehandlung zog man die Pflanzen je nach Wuchsform in den Versuchsgefässen zunächst bis zu einer Höhe von 3 bis 10 cm an und behandelte sie dann. Die Versuchstöpfe wurden dann unabgedeckt im Gewächshaus aufgestellt, wobei für wärmeliebende Arten heissere Bereiche (25 bis 40°C) und für solche gemässigter Klimate 15 bis 30°C bevorzugt wurden. Die Versuchsperiode erstreckte sich über 3 bis 6 Wochen. Während dieser Zeit wurden die Pflanzen gepflegt, und ihre Reaktion auf die einzelner Behandlungen wurde ausgewertet. Die Auswertung der Versuche wurde nach einer Skala von 0 bis 100 vorgenommen. Dabei bedeutet 0 keine Schädigung oder normales Wachstum und 100 kein Aufgang der Pflanzen bzw. Völlige Zerstörung zumindest der oberirdischen Sprossteile. Die in den Versuchen getesteten Pflanzenarten sind in Tabelle 1 aufgeführt. Die Versuchsergebnisse sind Gegenstand der Tabellen 2 - 9.

Tabelle 1

Liste der Pflanzennamen

| Botanischer Name | Abkürzung in Tabellen | Deutscher Name |
|---|---|---|
| Allium cepa | — | Küchenzwiebel |
| Alopecurus myosuroides | Alopec. myos. | Ackerfuchsschwanz |
| Arachys hypogaea | Arachys hyp. | Erdnuss |
| Avena fatua | — | Flughafer |
| Beta vulgaris | — | Zuckerrübe |
| Brassica napus | — | Raps |
| Bromus spp. | — | Trespe-Arten |
| Cassia spp. | — | |
| Centaurea cyanus | Cent. cyan. | Kornblume |
| Chenopodium album | Chenopod. alb. | Weisser Gänserfuss |
| Datura stramonium | — | Gemeiner Stechapfel |
| Daucus carota | — | Möhre |
| Desmodium tortuosum | | |
| Glycine max | — | Sojabohne |
| Lolium multiflorum | — | Ital. Raygras |
| Matricaria spp. | Matric. spp. | Kamillearten |
| Nicandra physaloides | Nicand. phys. | Giftbeere |
| Sesbania exaltata | Sesbania exalt. | Turibaum |
| Sinapis alba | — | Weisser Senf |
| Sorghum bicolor | Sorgh. bicol. | Mohrenhirse |
| Solanum nigrum | Solan. nigr. | Schwarzer Nachtschatten |
| Stellaria media | — | Vogelsternmiere |
| Triticum aestivum | Tritic. aest. | Weizen |
| Mercurials annua | Mercur. ann. | Einjähriges Bingelkraut |
| Oryza sativa | Oryza sat. | Reis |

Tabelle 2

Herbizide Wirkung auf Lolium multiflorum bei Vorauflaufanwendung im Gewächshaus

| Wirkstoff Nr. | $R^1$ | $R^2$ | $R^3$ | $R^4$ | Schädigung (%) bei einer Aufwandmenge von 3,0 kg a.S./ha |
|---|---|---|---|---|---|
| 9 | $CH_3OCH_2$ | H | $CH_3$ | 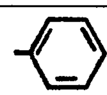 | 100 |
| 11 | $CH_3OCH_2$ | H | $CH_3$ | 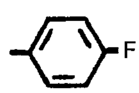 | 100 |
| 2 | $CH_3OCH_2$ | H | H | 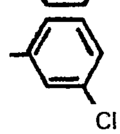 | 95 |
| 4 | $CH_3OCH_2$ | H | $CH_3$ | 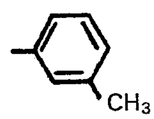 | 100 |

Tabelle 3

Nachauflaufwirkung auf Lolium multiflorum im Gewächshaus

| Wirkstoff Nr. | $R^1$ | $R^2$ | $R^3$ | $R^4$ | Schädigung (%) bei einer Aufwandmenge von 3,0 kg a.S./ha |
|---|---|---|---|---|---|
| 12 | $CH_3OCH_2$ | H | $CH_3$ | 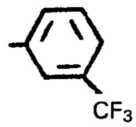 | 100 |

Tabelle 4

Selektive herbizide Wirkung bei Nachauflaufanwendung im Gewächshaus

| Wirkstoff Nr. | Formel | Aufwandmenge (kg a.S./ha) | Testpflanzen und Schädigung (%) Arachys hyp. | Glycine max | Sorgh. bicol. | Cassia spp. | Desmodium tort. | Nicand. phys. | Sesban. exalt. |
|---|---|---|---|---|---|---|---|---|---|
| 10 | 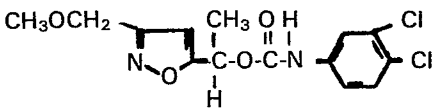 | 1,0 | 0 | 10 | 10 | 90 | 100 | 100 | 100 |
| | 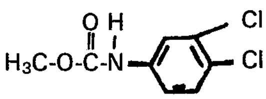 (US-A-3 116 995) | 1,0 | 10 | 20 | 15 | 0 | 100 | 20 | 60 |
| | 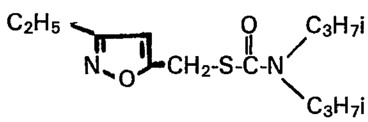 (DE-A-2 633 790) | 1,0 | 10 | 30 | 25 | 0 | 30 | 0 | 10 |

Tabelle 5

Selektive Wirkung bei Nachauflaufanwendung in Möhren im Gewächshaus

| Wirkstoff Nr. | Formel | Aufwandmenge (kg a.S./ha) | Testpflanzen und Schädigung (%) Daucus carota | Chenopod. alb. | Desmod. tort. | Sinapis alba |
|---|---|---|---|---|---|---|
| 12 | 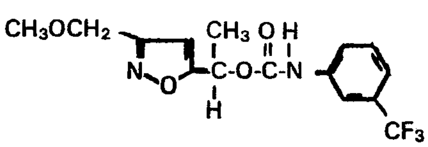 | 1,0 | 0 | 99 | 95 | 90 |
| | 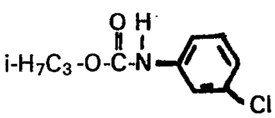 (US-A-2 695 225) | 1,0 | 10 | 20 | 10 | 80 |

Tabelle 6

Selektive herbizide Wirkung bei Nachauflaufanwendung im Gewächshaus

| Wirkstoff Nr. | Formel | Aufwandmenge (kg a.S./ha) | Testpflanzen und Schädigung (%) Arachys hyp. | Tritic. aest. | Cent. cyan | Matric. spp. | Nicand. phys. | Sinapis alba | Solan. nigr. |
|---|---|---|---|---|---|---|---|---|---|
| 11 | 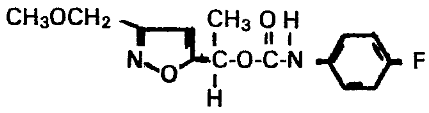 | 1,0 | 0 | 0 | 80 | 100 | 100 | 98 | 100 |
| | 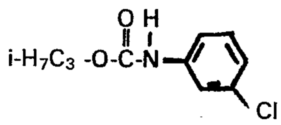 (US-A-2 695 225) | 1,0 | 10 | 80 | — | 0 | 0 | 80 | 50 |
| | 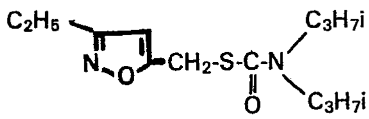 (DE-A- 2 633 790) | 1,0 | 10 | 70 | — | 0 | 0 | 20 | 70 |

Tabelle 7

Selektive Bekämpfung von unerwünschten Gräsern und breitblättrigen Unkräutern bei Nachauflaufanwendung im Gewächshaus

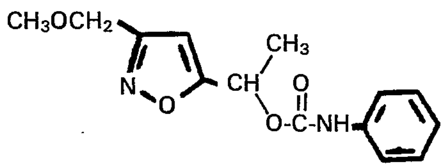

(Nr. 9)

| Testpflanzen | Schädigung (%) bei 2,0 kg a.S./ha |
|---|---|
| Allium cepa | 0 |
| Beta vulgaris | 0 |
| Alopecurus myosuroides | 90 |
| Avena fatua | 80 |
| Bromus spp. | 90 |
| Centaurea cyanus | 80 |
| Datura stramonium | 80 |
| Sinapis alba | 100 |
| Stellaria media | 90 |

Tabelle 8

Selektive Bekämpfung von Chenopodium in Zuckerrüben und anderen Kulturen bei Nachauflaufanwendung im Gewächshaus

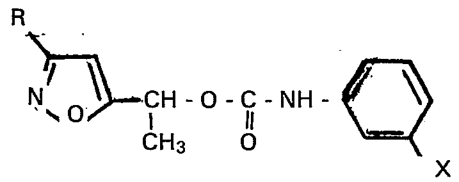

| Wirkstoff Nr. | X | R | kg/ha a.S. | Testpflanzen und Schädigung<br>Arachys hyp. | Beta vulg. | Oryza sat. | Chenop. album |
|---|---|---|---|---|---|---|---|
| 4 | $CH_3$ | $-CH_2-O-CH_3$ | 1,0 | 0 | 0 | 10 | 100 |
| | | | 2,0 | 0 | 0 | 10 | 100 |
| 8 | $CF_3$ | $-COOC_2H_5$ | 0,5 | 0 | 0 | 0 | 100 |
| | | | 1,0 | 0 | 10 | 0 | 100 |

* = keine Schädigung
100 = Pflanzen abgestorben

Tabelle 9

Selektive Unkrautbekämpfung bei Nachauflaufanwendung im Gewächshaus

| Wirkstoff Nr. | Struktur | kg/ha a.S. | Testpflanzen und Schädigung (%)<br>Arachys hyp. | Glycine max | Sorgh. bicol. | Chenop. album | Datura stram. | Desmod. tort. | Mercur. ann. |
|---|---|---|---|---|---|---|---|---|---|
| 3 | 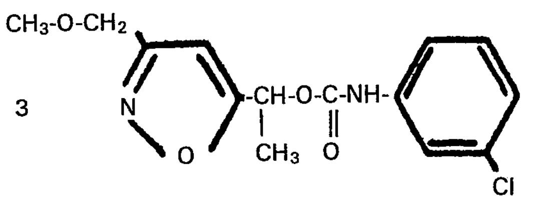 | 0,5 | 0 | 0 | 0 | 100 | 100 | 100 | 90 |

0 = keine Schädigung
100 = Pflanzen abgestorben

In Anbetracht der Vielseitigkeit der Applikationsmethoden können die erfindungsgemässen Mittel oder diese enthaltende Mischungen ausser bei den in den Tabellen aufgeführten Nutzpflanzen noch in einer weiteren grossen Zahl von Kulturen zur Beseitigung unerwünschten Pflanzenwuchses eingesetzt werden.

Im einzelnen seien folgende Nutzpflanzen genannt:

| Botanischer Name | Deutscher Name |
|---|---|
| Allium cepa | Küchenzwiebel |
| Ananas comosus | Ananas |
| Arachis hypogaea | Erdnuss |
| Asparagus officinalis | Spargel |
| Avena sativa | Hafer |
| Beta vulgaris spp. altissima | Zuckerrübe |
| Beta vulgaris spp. rapa | Futterrübe |
| Beta vulgaris spp. esculenta | Rote Rübe |
| Brassica napus var. napus | Raps |
| Brassica napus var. napobrassica | Kohlrübe |
| Brassica napus var. rapa | Weisse Rübe |
| Brassica rapa var. silvestris | Rübsen |
| Camellia sinensis | Teestrauch |
| Carthamus tinctorius | Saflor - Färberdistel |
| Carva illinoinensis | Pekannussbaum |
| Citrus limon | Zitrone |
| Citrus maxima | Pampelmuse |
| Citrus reticulata | Mandarine |
| Citrus sinensis | Apfelsine, Orange |
| Coffea arabica (Coffea canephora, Coffea liberica) | Kaffee |
| Cucumis melo | Melone |
| Cucumis sativus | Gurke |
| Cynodon dactylon | Bermudagras |
| Daucus carota | Möhre |
| Elaeis guineensis | Ölpalme |
| Fragaria vesca | Erdbeere |
| Glycine max | Sojabohne |
| Gossypium hirsutum (Gossypium arboreum Gossypium herbaceum Gossypium vitifolium) | Baumwolle |
| Helianthus annuus | Sonnenblume |
| Helianthus tuberosus | Topinambur |
| Hevea brasiliensis | Parakautschukbaum |
| Hordeum vulgare | Gerste |
| Humulus lupulus | Hopfen |
| Ipomoea batatas | Süsskartoffeln |
| Juglans regia | Walnussbaum |
| Lactua sativa | Kopfsalat |
| Lens culinaris | Linse |
| Linum usitatissimum | Faserlein |
| Lycopersiocon lyocopersicum | Tomate |
| Malus spp. | Apfel |
| Manihot esculenta | Maniok |
| Medicago sativa | Luzerne |
| Mentha piperita | Pfefferminze |
| Musa spp. | Obst- u. Mehlbanane |
| Nicotiana tabacum (N. rustica) | Tabak |
| Olea europaea | Ölbaum |
| Oryza sativa | Reis |
| Panicum miliaceum | Rispenhirse |
| Phaseolus lunatus | Mondbohne |
| Phaseolus mungo | Urdbohne |
| Phaseolus vulgaris | Buschbohnen |
| Pennisetum glaucum | Perl- oder Rohrkolbenhirse |

| Botanischer Name | Deutscher Name |
|---|---|
| Petroselinim crispum spp. tuberosum | Wurzelpetersilie |
| Picea abies | Rotfichte |
| Abies alba | Weisstanne |
| Pinus spp. | Kiefer |
| Pisum sativum | Gartenerbse |
| Prunus avium | Süsskirsche |
| Prunus domestica | Pflaume |
| Prunus dulcis | Mandelbaum |
| Prunus persica | Pfirsich |
| Pyrus communis | Birne |
| Ribes sylvestre | Rote Johannisbeere |
| Ribes uva-crispa | Stachelbeere |
| Ricinus communis | Rizinus |
| Saccharum officinarum | Zuckerrohr |
| Secale cereale | Roggen |
| Sesamum indicum | Sesam |
| Solanum tuberosum | Kartoffel |
| Sorghum bicolor (s. vulgare) | Mohrenhirse |
| Sorghum dochna | Zuckerhirse |
| Spinacia oleracea | Spinat |
| Theobroma cacao | Kakaobaum |
| Trifolium pratense | Rotklee |
| Triticum aestivum | Weizen |
| Vaccinium carymbosum | Kulturheidelbeere |
| Vaccinium vitis-idaea | Preisselbeere |
| Vicia faba | Pferdebohnen |
| Vigna sinensis (V. unguiculata) | Kuhbohne |
| Vitis vinifera | Weinrebe |
| Zea mays | Mais |

Die neuen 1,2-Oxazolylalkylcarbamate können sowohl unter sich als auch mit zahlreichen Vertretern anderer herbizider oder wachstumsregulierender Wirkstoffgruppen gemischt und gemeinsam ausgebracht werden. Beispielsweise kommen als Mischungspartner Diazine, N-Phenyl-carbamate, Thiolcarbamate, Diurethane, Halogencarbonsäuren, Phenoxyfettsäuren, Triazine, Amide, Harnstoffe, Diphenylether, Triazone, Uracile, Benzofuranderivate und andere in Betracht. Solche Kombinationen dienen zur Verbreiterung des Wirkungsspektrums und erzielen zuweilen synergistische Effekte. Eine Reihe von Wirkstoffen, welche zusammen mit den neuen Verbindungen für verschiedenste Anwendungsbereiche sinnvolle Mischungen ergeben, werden beispielhaft aufgeführt:

5-Amino-4-chlor-2-phenyl-3(2H)-pyridazinon
5-Amino-4-brom-2-phenyl-3(2H)-pyridazinon
5-Amino-4-chlor-2-cyclohexyl-3(2H)-pyridazinon
5-Amino-4-brom-2-cyclohexyl-3(2H)-pyridazinon
5-Methylamino-4-chlor-2-(3-trifluormethylphenyl)-3(2H)-pyridazinon
5-Methylamino-4-chlor-2-(3-α,α,β,β-tetrafluorethoxyphenyl)-3(2H)-pyridazinon
5-Dimethylamino-4-chlor-2-phenyl-3(2H)-pyridazinon
4,5-Dimethoxy-2-phenyl-3(2H)-pyridazinon
4,5-Dimethoxy-2-cyclohexyl-3(2H)-pyridazinon

4,5-Dimethoxy-2-(3-trifluormethylphenyl)-3(2H)-pyridazinon
5-Methoxy-4-chlor-2-(3-trifluormethylphenyl)-3(2H)-pyridazinon
5-Amino-4-brom-2-(3-methylphenyl)-3(2H)-pyridazinon

3-(1-Methylethyl)-1H-2,1,3-benzothiadiazin-4(3H)-on-2,2-dioxid und Salze
3-(1-Methylethyl)-8-chlor-1H-2,1,3-benzothiadiazin-4(3H)-on-2,2-dioxid und Salze
3-(1-Methylethyl)-8-fluor-1H-2,1,3-benzothiadiazin-4(3H)-on-2,2-dioxid und Salze
3-(1-Methylethyl)-8-methyl-1H-2,1,3-benzothiadiazin-4(3H)-on-2,2-dioxid und Salze

1-Methoxymethyl-3-(1-methylethyl)-2,1,3-benzothiadiazin-4(3H)-on-2,2-dioxid
1-Methoxymethyl-8-chlor-3-(1-methylethyl)-2,1,3-benzothiadiazin-4(3H)-on-2,2-dioxid
1-Methoxymethyl-8-fluor-3-(1-methylethyl)-2,1,3-benzothiadiazin-4(3H)-on-2,2-dioxid
1-Cyan-8-chlor-3-(1-methylethyl)-2,1,3-benzothiadiazin-4(3H)-on-2,2-dioxid
1-Cyan-8-fluor-3-(1-methylethyl)-2,1,3-benzothiadiazin-4(3H)-on-2,2-dioxid
1-Cyan-8-methyl-3-(1-methylethyl)-2,1,3-benzothiadiazin-4(3H)-on-2,2-dioxid
1-Cyan-3-(1-methylethyl)-2,1,3-benzothiadiazin-4(3H)-on-2,2-dioxid
1-Azidomethyl-3-(1-methylethyl)-2,1,3-benzothiadiazin-4(3H)-on-2,2-dioxid
3-(1-Methylethyl)-1H-Pyridino-[3,2-e]-2,1,3-thiadiazin-(4)-on-2,2-dioxid

N-(1-Ethylpropyl)-2,6-dinitro-3,4-dimethylanilin
N-(1-Methylethyl)-N-ethyl-2,6-dinitro-4-trifluormethyl-anilin
N-n-Propyl-N-β-chlorethyl-2,6-dinitro-4-trifluormethyl-anilin
N-n-Propyl-N-cyclopropylmethyl-2,6-dinitro-4-trifluormethyl-anilin
N-Bis-(n-propyl)-2,6-dinitro-3-amino-4-trifluormethylanilin
N-Bis-(n-propyl)-2,6-dinitro-4-methyl-anilin
N-Bis-(n-propyl)-2,6-dinitro-4-methylsulfonyl-anilin
N-Bis-(n-propyl)-2,6-dinitro-4-aminosulfonyl-anilin
Bis-(β-chlorethyl)-2,6-dinitro-4-methyl-anilin
N-Ethyl-N-(2-methylallyl)-2,6-dinitro-4-trifluormethyl-anilin

N-Methylcarbaminsäure-3,4-dichlorbenzylester
N-Methylcarbaminsäure-2,6-di-tert-butyl-4-methylphenyl-ester
N-Phenylcarbaminsäure-isopropylester
N-3-Fluorphenylcarbaminsäure-3-methoxypropyl-2-ester
N-3-Chlorphenylcarbaminsäure-isopropylester
N-3-Chlorphenylcarbaminsäure-butin-1-yl-3-ester
N-3-Chlorphenylcarbaminsäure-4-chlor-butin-2-yl-1-ester
N-3,4-Dichlorphenylcarbaminsäure-methylester
N-(4-Amino-benzolsulfonyl)-carbaminsäure-methylester
O-(N-Phenylcarbamoyl)-propanonoxim
N-Ethyl-2-(phenylcarbamoyl)-oxypropionsäureamid
3'-N-Isopropyl-carbamoyloxy-propionanilid

Ethyl-N-[3-(N'-phenylcarbamoyloxy)-phenyl]-carbamat
Methyl-N-[3-(N'-methyl-N'-phenylcarbamoyloxy)-phenyl]-carbamat
Isopropyl-N-[3-(N'-ethyl-N'-phenylcarbamoyloxy)-phenyl)-carbamat
Methyl-N-[3-(N'-3-methylphenylcarbamoyloxy)-phenyl]-carbamat
Methyl-N-[3-(N'-4-fluorphenylcarbamoyloxy)-phenyl]-carbamat
Methyl-N-[3-(N'-3-chlor-4-fluorphenylcarbamoyloxy)-phenyl]-carbamat

Ethyl-N-[3-(N'-chlor-4-fluorphenylcarbamoyloxy)-phenyl]-carbamat
Ethyl-N-[3-(N'-3,4-difluorphenylcarbamoyloxy)-phenyl]-carbamat
Methyl-N-[3-(N'-3,4-difluorphenylcarbamoyloxy)-phenyl]-carbamat
N-3-(4-Fluorphenoxycarbonylamino)-phenyl-carbaminsäure-methylester
N-3-(2-Methylphenoxycarbonylamino)-phenyl-carbaminsäure-ethylester
N-3-(4-Fluorphenoxycarbonylamino)-phenyl-thiolcarbaminsäure-methylester
N-3-(2,4,5-Trimethylphenoxycarbonylamino)-phenyl-thiolcarbaminsäure-methylester
N-3-(Phenoxycarbonylamino)-phenyl-thiolcarbaminsäure-methylester
N,N-Diethyl-thiolcarbaminsäure-p-chlorbenzylester
N,N-Di-n-propyl-thiolcarbaminsäure-ethylester
N,N-Di-n-propyl-thiolcarbaminsäure-n-propylester
N,N-Di-isopropyl-thiolcarbaminsäure-2,3-dichlorallylester
N,N-Di-isopropyl-thiolcarbaminsäure-2,3,3-trichlorallylester
N,N-Di-isopropyl-thiolcarbaminsäure-3-methyl-5-isoxazolyl-methylester
N,N-Di-isopropyl-thiolcarbaminsäure-3-ethyl-5-isoxazolyl-methylester
N,N-Di-sec.butyl-thiolcarbaminsäure-ethylester
N,N-Di-sec.butyl-thiolcarbaminsäure-benzylester
N-Ethyl-N-cyclohexyl-thiolcarbaminsäure-ethylester
N-Ethyl-N-bicyclo-[2.2.1]-heptyl-thiolcarbaminsäureethylester

S-(2,3-Dichlorallyl)-(2,2,4-trimethyl-azetidin)-1-carbothiolat
S-(2,3,3-Trichlorallyl)-(2,2,4-trimethyl-azetidin)-1-carbothiolat
S-Ethyl-hexahydro-1-H-azepin-1-carbothiolat
S-Benzyl-(3-methyl-hexahydro-1-H-azepin-1)-carbothiolat
S-Benzyl-(2,3-dimethylhexahydro-1-H-azepin-1)-carbothiolat
S-Ethyl-(3-methylhexahydro-1-H-azepin-1)-carbothiolat
N-Ethyl-N-n-butyl-thiolcarbaminsäure-n-propylester
N,N-Dimethyl-dithiocarbaminsäure-2-chlorallylester
N-Methyl-dithiocarbaminsäure-Natriumsalz
Trichloressigsäure-Natriumsalz
α,α-Dichlorpropionsäure-Natriumsalz
α,α-Dichlorbuttersäure-Natriumsalz
α,α,β,β-Tetrafluorpropionsäure-Natriumsalz

α-Methyl-α,β-dichlorpropionsäure-Natriumsalz
α-Chlor-β-(4-chlorphenyl)-propionsäure-methylester
α,β-Dichlor-β-phenylpropionsäure-methylester
Benzamido-oxy-essigsäure

2,3,5-Trijodbenzoesäure (Salze, Ester, Amide)
2,3,6-Trichlorbenzoesäure (Salze, Ester, Amide)
2,3,5,6-Tetrachlorbenzoesäure (Salze, Ester, Amide)
2-Methoxy-3,6-dichlorbenzoesäure (Salze, Ester, Amide)
2-Methoxy-3,5,6-trichlorbenzoesäure (Salze, Ester, Amide)
3-Amino-2,5,6-trichlorbenzoesäure (Salze, Ester, Amide)
O,S-Dimethyl-tetrachlor-thioterephthalat
Dimethyl-2,3,5,6-tetrachlor-terephthalat
Dinatrium-3,6-endoxohexahydro-phthalat
4-Amino-3,5,6-trichlor-picolinsäure (Salze)
2-Cyan-3-(N-methyl-N-phenyl)-amino-acrylsäure-ethylester
2-[4-(4'-Chlorphenoxy)-phenoxy]-propionsäureisobutylester
2-[4-(2',4'-Dichlorphenoxy)-phenoxy]-propionsäuremethylester
2-[4-(4'-Trifluormethylphenoxy)-phenoxy]-propionsäure-methylester
2-[4-(2'-Chlor-4'-trifluorphenoxy)-phenoxy]-propionsäure-Natriumsalz
2-[4-(3',5'-Dichlorpyridyl-2-oxy)-phenoxy]-propionsäure-Natriumsalz

2-(N-Benzoyl-3,4-dichlorphenylamino)-propionsäureethylester
2-(N-Benzoyl-3-chlor-4-fluorphenylamino)-propionsäure-methylester
2-(N-Benzoyl-3-chlor-4-fluorphenylamino)-propionsäureisopropylester

2-Chlor-4-ethylamino-6-isopropylamino-1,3,5-tri azin
2-Chlor-4-ethylamino-6-(amino-2'-propionitril)-1,3,5-triazin
2-Chlor-4-ethylamino-6-2-methoxypropyl-2-amino-1,3,5-triazin
2-Chlor-4-ethylamino-6-butin-1-yl-amino-1,3,5-triazin
2-Chlor-4,6-bisethylamino-1,3,5-triazin
2-Chlor-4,6-bisisopropylamino-1,3,5-triazin
2-Chlor-4-isopropylamino-6-cyclopropylamino-1,3,5-triazin

2-Azido-4-methylamino-6-isopropylamino-1,3,5-triazin
2-Methylthio-4-ethylamino-6-isopropylamino-1,3,5-triazin
2-Methylthio-4-ethylamino-6-tert.butylamino-1,3,5-triazin
2-Methylthio-4,6-bisethylamino-1,3,5-triazin
2-Methylthio-4,6-bisisopropylamino-1,3,5-triazin

2-Methoxy-4-ethylamino-6-isopropylamino-1,3,5-triazin
2-Methoxy-4,6-bisethylamino-1,3,5-triazin
2-Methoxy-4,6-bisisopropylamino-1,3,5-triazin
4-Amino-6-tert.butyl-3-methylthio-4,5-dihydro-1,2,4-triazin-5-on
4-Amino-6-phenyl-3-methyl-4,5-dihydro-1,2,4-triazin-5-on
4-Isobutylidenamino-6-tert.butyl-3-methylthio-4,5-dihydro-1,2,4-triazin-5-on
1-Methyl-3-cyclohexyl-6-dimethylamino-1,3,5-triazin-2,4-dion
3-tert.Butyl-5-chlor-6-methyluracil
3-tert.Butyl-5-brom-6-methyluracil
3-Isopropyl-5-brom-6-methyluracil
3-sec.Butyl-5-brom-6-methyluracil
3-(2-Tetrahydropyranyl)-5-chlor-6-methyluracil
3-(2-Tetrahydropyranyl)5,6-trimethylenuracil
3-Cyclohexyl-5,6-trimethylenuracil

2-Methyl-4-(3'-trifluormethylphenyl)-tetrahydro-1,2,4-oxadiazin-3,5-dion
2-Methyl-4-(4'-fluorphenyl)-tetrahydro-1,2,4-oxadiazin-3,5-dion
3-Amino-1,2,4-triazol
1-Allyloxy-1-(4-bromphenyl)-2-[1',2',4'-triazolyl-(1')]-ethan (Salze)
[-1-(1,2,4-Triazolyl-(1')][-1(4'-chlorphenoxy)]-3,3-dimethylbutan-2-on
N,N-Diallylchloracetamid
N-Isopropyl-2-chloracetanilid
N-(Butin-1-yl-3)-2-chloracetanilid

2-Methyl-6-ethyl-N-(propargyl)-2-chloracetanilid
2-Methyl-6-ethyl-N-(äthoxymethyl)-2-chloracetanilid
2-Methyl-6-ethyl-N-(2-methoxy-1-methyläthyl)-2-chloracetanilid
2-Methyl-6-ethyl-N-(isopropoxycarbonyläthyl)-2-chloracetanilid
2-Methyl-6-ethyl-N-(4-methoxypyrazol-1-yl-methyl)-2-chloracetanilid
2-Methyl-6-ethyl-N-(pyrazol-1-yl-methyl)-2-chloracetanilid
2,6-Dimethyl-N-(pyrazon-1-yl-methyl)-2-chloracetanilid
2,6-Dimethyl-N-(4-methylpyrazol-1-yl-methyl)-2-chloracetanilid
2,6-Dimethyl-N-(1,2,4-triazol-1-yl-methyl)-2-chloracetanilid
2,6-Dimethyl-N-(3,5-dimethylpyrazol-1-yl-methyl)-2-chloracetanilid
2,6-Dimethyl-N-(1,3-dioxolan-2-yl-methyl)-2-chloracetanilid
2,6-Dimethyl-N-(2-methoxyethyl)-2-chloracetanilid
2,6-Dimethyl-N-(isobutoxymethyl)-2-chloracetanilid
2,6-Diethyl-N-(methoxymethyl)-2-chloracetanilid
2,6-Diethyl-N-(n-butoxymethyl)-2-chloracetanilid
2,6-Diethyl-N-(ethoxycarbonylmethyl)-2-chloracetanilid
2,3,6-Trimethyl-N-(pyrazol-1-yl-methyl)-2-chloracetanilid
2,3-Dimethyl-N-(isopropyl)-2-chloracetanilid

2-(2-Methyl-4-chlorphenoxy-N-methoxy-acetamid
2-(α-Naphthoxy)-N,N-diethylpropionamid
2,2-Diphenyl-N,N-dimethylacetamid

α-(3,4,5-Tribrompyrazol-1-yl)-N,N-dimethylpropionamid
n-(1,1-Dimethylpropinyl)-3,5-dichlorbenzamid
N-1-Naphthylphthalamidsäure
Propionsäure-3,4-dichloranilid
Methacrylsäure-3,4-dichloranilid
2-Methylpentancarbonsäure-3,4-dichloranilid
N-2,4-Dimethyl-5-(trifluormethyl)-sulfonylamino-phenylacetamid
N-4-Methyl-5-(trifluormethyl)-sulfonylamino-phenylacetamid
2-Propionyl-amino-4-methyl-5-chlor-thiazol
O-(Methylsulfonyl)-glykolsäure-N-ethoxymethyl-2,6-dimethyl-anilid
O-(Methylaminosulfonyl)-glykolsäure-N-isopropyl-anilid
O-(i-Propylaminosulfonyl)-glykolsäure-N-butin-1-yl-3-anilid
O-(Methylaminosulfonyl)-glykolsäure-hexamethylenimid
2,6-Dichlor-thiobenzamid
2,6-Dichlorbenzonitril
3,5-Dibrom-4-hydroxy-benzonitril (Salze)
3,5-Dijod-4-hydroxy-benzonitril (Salze)
3,5-Dibrom-4-hydroxy-O-2,4-dinitrophenylbenzaldoxim (Salze)
3,5-Dibrom-4-hydroxy-O-2-cyan-4-nitrophenylbenzaldoxim (Salze)
Pentachlorphenyl-Natriumsalz
2,5-Dichlorphenyl-4′-nitrophenylether
2,4,6-Trichlorphenyl-4′-nitrophenylether
2-Fluor-4,6-dichlorphenyl-4′-nitrophenylether
2-Chlor-4-trifluormethylphenyl-4′-nitrophenylether
2,4′-Dinitro-4-trifluormethyl-diphenylether
2,4-Dichlorphenyl-3′-methoxy-4′-nitro-phenylether
2-Chlor-4-trifluormethylphenyl-3′-ethoxy-4′-nitro-phenylether
2-Chlor-4-trifluormethylphenyl-3′-carboxy-4′-nitro-phenylether (Salze)
2-Chlor-4-trifluormethylphenyl-3′-ethoxycarbonyl-4′-nitro-phenylether
2,4-Dichlorphenyl-3′-methoxycarbonyl-4′-nitro-phenylether
2-(3,4-Dichlorphenyl)-4-methyl-1,2,4-oxadiazolidin-3,5-dion
2-(3-tert.Butylcarbamoyloxy-phenyl)-4-methyl-1,2,4-oxadiazolidin-3,5-dion
2-(3-i-Propylcarbamoyloxy-phenyl)-4-methyl-1,2,4-oxadiazolidin-3,5-dion
2-Phenyl-3,1-benzoxazinon-(4)
(4-Bromphenyl)-3,4,5,9,10-pentaazatetracyclo-[5,4,1,$0^{2,6}$,$0^{8,11}$]-dodeca-3,9-dien
2-Ethoxy-2,3-dihydro-3,3-dimethyl-5-benzofuranyl-methan-sulfonat
2-Ethoxy-2,3-dihydro-3,3-dimethyl-5-benzofuranyl-dimethyl-aminosulfat
2-Ethoxy-2,3-dihydro-3,3-dimethyl-5-benzofuranyl-(N-methyl-N-acetyl)-aminosulfat
3,4-Dichlor-1,2-benzisothiazol
N-4-Chlorphenyl-allylbernsteinsäureimid
2-Methyl-4,6-dinitrophenol (Salze, Ester)
2-sec.Butyl-4,6-dinitrophenol (Salze, Ester)
2-sec.Butyl-4,6-dinitrophenol-acetat
2-tert.Butyl-4,6-dinitrophenol-acetat
2-tert.Butyl-4,6-dinitrophenol (Salze)
2-tert.Butyl-5-methyl-4,6-dinitrophenol (Salze)
2-tert.Butyl-5-methyl-4,6-dinitrophenol-acetat

2-sec.Amyl-4,6-dinitrophenol (Salze, Ester)
1-(α,α-Dimethylbenzyl)-3-(4-methylphenyl)-harnstoff
1-Phenyl-3-(2-methylcyclohexyl)-harnstoff
1-Phenyl-1-benzoyl-3,3-dimethyl-harnstoff
1-(4-Chlorphenyl)-1-benzoyl-3,3-dimethyl-harnstoff
1-(4-Chlorphenyl)-3,3-dimethyl-harnstoff
1-(4-Chlorphenyl)-3-methyl-3-butin-1-yl-3-harnstoff

1-(3,4-Dichlorphenyl)-3,3-dimethyl-harnstoff
1-(3,4-Dichlorphenyl)-1-benzoyl-3,3-dimethyl-harnstoff
1-(3,4-Dichlorphenyl)-3-methyl-3-n-butyl-harnstoff
1-(4-i-Propylphenyl)-3,3-dimethyl-harnstoff
1-(3-Trifluormethylphenyl)-3,3-dimethyl-harnstoff
1-(3-α,α,β,β-Tetrafluorethoxyphenyl)-3,3-dimethyl-harnstoff

1-(3-tert.Butylcarbamoyloxy-phenyl)-3,3-dimethyl-harnstoff
1-(3-Chlor-4-methylphenyl)-3,3-dimethyl-harnstoff
1-(3-Chlor-4-methoxyphenyl)-3,3-dimethyl-harnstoff
1-(3,5-Dichlor-4-methoxyphenyl)-3,3-dimethyl-harnstoff
1-[4-(4′-Chlorphenoxy)-Phenyl]-3,3-dimethyl-harnstoff
1-[4-(4′-Methoxyphenoxy)-phenyl]-3,3-dimethyl-harnstoff
1-Cyclooctyl-3,3-dimethyl-harnstoff
1-(Hexahydro-4,7-methanindan-5-yl)-3,3-dimethyl-harnstoff
1-[1- oder 2-(3a, 4, 5, 7, 7a-Hexahydro)-4,7-methanoindanyl]-3,3-dimethyl-harnstoff
1-(4-Fluorphenyl)-3-carboxymethoxy-3-dimethyl-harnstoff
1-Phenyl-3-methyl-3-methoxy-harnstoff
1-(4-Chlorphenyl)-3-methyl-3-methoxy-harnstoff
1-(4-Bromphenyl)-3-methyl-3-methoxy-harnstoff
1-(3,4-Dichlorphenyl)-3-methyl-3-methoxy-harnstoff
1-(3-Chlor-4-bromphenyl)-3-methyl-3-methoxy-harnstoff
1-(3-Chlor-4-isopropylphenyl)-3-methyl-3-methoxy-harnstoff
1-(3-Chlor-4-methoxyphenyl)-3-methyl-3-methoxy-harnstoff
1-(3-tert.Butylphenyl)-3-methyl-3-methoxy-harnstoff
1-(2-Benzthiazolyl)-1,3-dimethyl-harnstoff
1-(2-Benzthiazolyl)-3-methyl-harnstoff
1-(5-Trifluormethyl-1,3,4-thiadiazolyl)-1,3-dimethyl-harnstoff
1-(4-Benzyloxyphenyl)-3-methyl-3-methoxy-harnstoff
Imidazolidin-2-on-1-carbonsäure-iso-butylamid
1,2-Dimethyl-3,5-diphenylpyrazolium-methylsulfat
1,2,4-Trimethyl-3,5-diphenylpyrazolium-methylsulfat

1,2-Dimethyl-4-brom-3,5-diphenylpyrazolium-methylsulfat
1,3-Dimethyl-4-(3,4-dichlorbenzoyl)-5-[(4-methylphenylsulfonyl)-oxy]-pyrazol
2,3,5-Trichlor-pyridinol-(4)
1-Methyl-3-phenyl-5-(3'-trifluormethylphenyl)-pyridon-(4)
1-Methyl-4-phenyl-pyridiniumchlorid
1,1-Dimethylpyridiniumchlorid
3-Phenyl-4-hydroxy-6-chlorpyridazin
1,1'-Dimethyl-4,4'-dipyridylium-di-(methylsulfat)
1,1'-Di-(3,5-dimethylmorpholin-carbonylmethyl)-4,4'-dipyridylium-dichlorid
1,1'-Ethylen-2,2'-dipyridylium-dibromid
3-[1-(N-Ethoxyamino)-propyliden]-6-ethyl-3,4-dihydro-2H-pyran-2,4-dion
3-[1-(N-Allyloxyamino)-propyliden]-6-ethyl-3,4-dihydro-2H-pyran-2,4-dion
2-[1-(N-Allyloxyamino)-propyliden]-5,5-dimethylcyclohexan-1,3-dion (Salze)
2-[1-(N-Allyloxyamino)-butyliden]-5,5-dimethylcyclohexan-1,3-dion (Salze)
2-[1-(N-Allyloxyamino)-butyliden]-5,5-dimethyl-4-methoxycarbonyl-cyclohexan-1,3-dion (Salze)
2-Chlorphenoxyessigsäure (Salze, Ester, Amide)
4-Chlorphenoxyessigsäure (Salze, Ester, Amide)
2,4-Dichlorphenoxyessigsäure (Salze, Ester, Amide)
2,4,5-Trichlorphenoxyessigsäur (Salze, Ester, Amide)
2-Methyl-4-chlorphenoxyessigsäure (Salze, Ester, Amide)
3,5,6-Trichlor-2-pyridinyl-oxyessigsäure (Salze, Ester, Amide)

α-Naphthoxyessigsäuremethylester
2-(2-Methylphenoxy)-propionsäure (Salze, Ester, Amide)
2-(4-Chlorphenoxy)-propionsäure (Salze, Ester, Amide)
2-(2,4-Dichlorphenoxy)-propionsäure (Salze, Ester, Amide)
2-(2,4,5,-Trichlorphenoxy)-propionsäure (Salze, Ester, Amide)
2-(2-Methyl-4-chlorphenoxy)-propionsäure (Salze, Ester, Amide)
4-(2,4-Dichlorphenoxy)-buttersäure (Salze, Ester, Amide)
4-(2-Methyl-4-chlorphenoxy)-buttersäure (Salze, Ester, Amide)
Cyclohexyl-3-(2,4-dichlorphenoxy)-acrylat
9-Hydroxyfluoren-carbonsäure-(9) (Salze, Ester)
2,3,6-Trichlorphenyl-essigsäure (Salze, Ester)
4-Chlor-2-oxo-benzothiazolin-3-yl-essigsäure (Salze, Ester)

Gibellerinsäure (Salze)
Dinatrium-methylarsonat
Mononatriumsalz der Methylarsonsäure
N-Phosphon-methyl-glycin (Salze)
N,N-Bis-(phosphonmethyl)-glycin (Salze)
2-Chlorethanphosphonsäure-2-chlorethylester
Ammonium-ethyl-carbamoyl-phosphonat
Di-n-butyl-1-n.butylamino-cyclohexyl-phosphonat
Trithiobutylphosphit
O,O-Diisopropyl-5-(2-benzosulfonylamino-ethyl)-phosphordithioat
2,3-Dihydro-5,6-dimethyl-1,4-dithiin-1,1,4,4-tetraoxid
5-tert.Butyl-3-(2,4-dichlor-5-isopropoxyphenyl)-1,3,4-oxadiazolon-(2)
4,5-Dichlor-2-trifluormethyl-benzimidazol (Salze)
1,2,3,6-Tetrahydropyridazin-3,6-dion(Salze)
Bernsteinsäure-mono-N-dimethylhydrazid (Salze)
(2-Chlorethyl)-trimethyl-ammoniumchlorid
(2-Methyl-4-phenylsulfonyl)-trifluormethansulfonanilid
1,1-Dimethyl-4,6-diisopropyl-5-indanylethylketon
2-[1-(2,5-Dimethylphenyl)-ethylsulfonyl]-pyridin-N-oxid
Natriumchlorat
Ammoniumrhodanid
Calciumcyanamid

Ausserdem ist es nützlich, die erfindungsgemässen Verbindungen allein oder in Kombination mit anderen Herbiziden auch noch mit weiteren Pflanzenschutzmitteln gemischt gemeinsam auszubringen, beispielsweise mit Mitteln zur Bekämpfung von Schädlingen oder phytopathogenen Pilzen bzw. Bakterien. Von Interesse ist ferner die Mischbarkeit mit Mineralstofflösungen, welche zur Behebung von Ernährungs- oder Spurenelementmängeln eingesetzt werden. Zu den Einzelwirkstoffen oder Mischungen können auch Öle verschiedenen Typs, Ölkonzentrate, Netz- oder Haftmittel sowie Antischaummittel zugesetzt werden.

**Patentansprüche für die Vertragsstaaten:**
BE, CH, DE, Fr, GB, IT, LI, NL, SE

1. 1,2-Oxazolylalkylcarbamate der Formel I

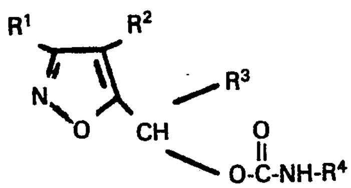

I

in der
$R^1$ ein Alkoxyalkylrest mit bis zu 3 Kohlenstoffatomen oder ein Alkoxycarbonylrest mit bis zu 5 Kohlenstoffatomen,
$R^2$ Wasserstoff oder ein Alkylrest mit bis zu 3 Kohlenstoffatomen, der durch Halogen substituiert sein kann,
$R^3$ Wasserstoff oder ein Alkylrest mit bis zu 3 Kohlenstoffatomen und
$R^4$ ein Phenylrest, der durch Halogen, Nitro, Cyano oder Alkyl-, Alkoxy-. Halogenalkyl-, Halogenalkoxy-, Alkanoyl- oder Alkoxycarbonylgruppen mit bis zu 5 Kohlenstoffatomen oder Cycloalkylgruppen mit bis zu 6 Kohlenstoffatomen substituiert sein kann,
sind.

2. 1-(3-Methoxymethyl-1,2-oxazolyl-5)-ethyl-N-(3-chlorphenyl)-carbamat.

3. 1-(3-Methoxymethyl-1,2-oxazolyl-5)-ethyl-N-(3-methylphenyl)-carbamat.

4. 1-(3-Methoxymethyl-1,2-oxazolyl-5)-ethyl-N-(3-trifluormethylphenyl)-carbamat.

5. Verfahren zur Herstellung der 1,2-Oxazolylalkylcarbamate der Formel I gemäss Anspruch 1, dadurch gekennzeichnet, dass man 5-Hydroxymethyl-1,2-oxazole der Formel II

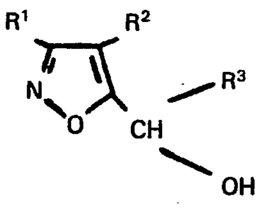

II

in der $R^1$, $R^2$ und $R^3$ die im Anspruch 1 genannten Bedeutungen haben, mit Isocyanaten der Formel III

$$OCN - R^4 \qquad III$$

in der $R^4$ die im Anspruch 1 genannten Bedeutungen hat, gegebenenfalls in Anwesenheit eines Verdünnungsmittels bei einer Temperatur zwischen —50 und +150°C umsetzt.

6. Herbizid, enthaltend mindestens ein 1,2-Oxazolylalkylcarbamat der Formel I gemäss Anspruch 1.

7. Verwendung eines 1,2-Oxazolylalkylcarbamats der Formel I gemäss Anspruch 1 als Herbizid.

8. Verfahren zur Bekämpfung unerwünschten Pflanzenwuchses, dadurch gekennzeichnet, dass man ein Herbizid, enthaltend ein 1,2-Oxazolylalkylcarbamat der Formel I gemäss Anspruch 1 verwendet.

**Patentansprüche für den Vertragsstaat:** AT

1. Herbizid, enthaltend ein 1,2-Oxazolylalkylcarbamat der Formel I

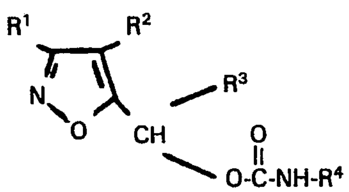

I

in der

$R^1$ ein Alkoxyalkylrest mit bis zu 3 Kohlenstoffatomen oder ein Alkoxycarbonylrest mit bis zu 5 Kohlenstoffatomen,

$R^2$ Wasserstoff oder ein Alkylrest mit bis zu 3 Kohlenstoffatomen, der durch Halogen substituiert sein kann,

$R^3$ Wasserstoff oder ein Alkylrest mit bis zu 3 Kohlenstoffatomen und

$R^4$ ein Phenylrest, der durch Halogen, Nitro, Cyano oder Alkyl-, Alkoxy-. Halogenalkyl-, Halogenalkoxy-, Alkanoyl- oder Alkoxycarbonylgruppen mit bis zu 5 Kohlenstoffatomen oder Cycloalkylgruppen mit bis zu 6 Kohlenstoffatomen substituiert sein kann,

sind, als Wirkstoff.

2. Herbizid, enthaltend 1-(3-Methoxymethyl-1,2-oxazolyl-5)-ethyl-N-(3-chlorphenyl)-carbamat als Wirkstoff.

3. Herbizid, enthaltend 1-(3-Methoxymethyl-1,2-oxazolyl-5)-ethyl-N-(3-methylphenyl)-carbamat als Wirkstoff.

4. Herbizid, enthaltend 1-(3-Methoxymethyl-1,2-oxazolyl-5)-ethyl-N-(3-trifluormethylphenyl)-carbamat als Wirkstoff.

5. Verfahren zur Herstellung der 1,2-Oxazolylalkylcarbamate der Formel I gemäss Anspruch 1, dadurch gekennzeichnet, dass man 5-Hydroxymethyl-1,2-oxazole der Formel II

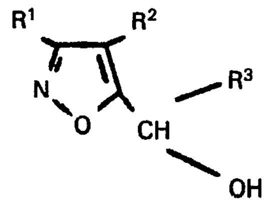

II

in der $R^1$, $R^2$ und $R^3$ die im Anspruch 1 genannten Bedeutungen haben, mit Isocyanaten der Formel III

$$OCN - R^4 \qquad III$$

in der $R^4$ die im Anspruch 1 genannten Bedeutungen hat, gegebenenfalls in Anwesenheit eines Verdünnungsmittels bei einer Temperatur zwischen —50 und +150°C umsetzt.

**Claims for the Contracting States:**
BE, CH, LI, DE, FR, GB, IT, LU, NL, SE

1. A 1,2-oxazolyl alkyl carbamate of the formula I

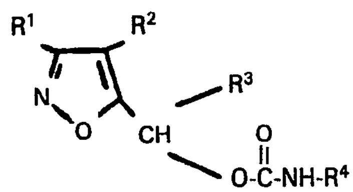

I

where $R^1$ is alkoxyalkyl of a maximum of 3 carbon atoms, or alkoxycarbonyl of a maximum of 5 carbon atoms, $R^2$ denotes hydrogen, or unsubstituted or halogen-substituted alkyl of a maximum of 3 carbon atoms, $R^3$ denotes hydrogen, or alkyl of a maximum of 3 carbon atoms, and $R^4$ denotes phenyl which is unsubstituted or substituted by halogen, nitro, cyano, or alkyl, alkoxy, haloalkyl, haloalkoxy, alkanoyl or alkoxycarbonyl of a maximum of 5 carbon atoms, or by cycloalkyl of a maximum of 6 carbon atoms.

2. 1-(3-Methoxymethyl-1,2-oxazol-5-yl)-ethyl-N-(3-chlorophenyl)-carbamate.

3. 1-(3-Methoxymethyl-1,2-oxazol-5-yl)-ethyl-N-(3-methylphenyl)-carbamate.

4. 1-(3-Methoxymethyl-1,2-oxazol-5-yl)-ethyl-N-(3-trifluoromethylphenyl)-carbamate.

5. A process for the manufacture of a 1,2-oxazolyl alkyl carbamate of the formula I as claimed in claim 1, wherein a 5-hydroxymethyl-1,2-oxazole of the formula II

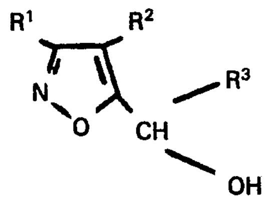

II

where $R^1$, $R^2$ and $R^3$ have the meanings given in claim 1, is reacted with an isocyanate of the formula III

$$OCN - R^4 \qquad III$$

where $R^4$ has the meanings given in claim 1, in the presence or absence of a diluent and at from —50° to +150°C.

6. A herbicide containing at least one 1,2-oxazolyl alkyl carbamate of the formula I as claimed in claim 1.

7. The use of a 1,2-oxazolyl alkyl carbamate of the formula I as claimed in claim 1 as a herbicide.

8. A process for combating the growth of unwanted plants, wherein a herbicide is used which contains a 1,2-oxazolyl alkyl carbamate of the formula I as claimed in claim 1.

**Claims for the Contracting State:** AT

1. A herbicide containing, as active ingredient, a 1,2-oxazolyl alkyl carbamate of the formula I

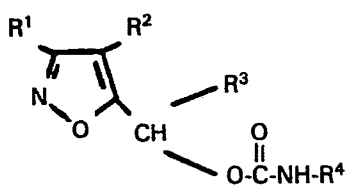

I

where $R^1$ is alkoxyalkyl of a maximum of 3 carbon atoms, or alkoxycarbonyl of a maximum of 5 carbon atoms, $R^2$ denotes hydrogen, or unsubstituted or halogen-substituted alkyl of a maximum of 3 carbon atoms, $R^3$ denotes hydrogen, or alkyl of a maximum of 3 carbon atoms, and $R^4$ denotes phenyl which is unsubstituted or substituted by halogen, nitro, cyano, or alkyl, alkoxy, haloalkyl, haloalkoxy, alkanoyl or alkoxycarbonyl of a maximum of 5 carbon atoms, or by cycloalkyl of a maximum of 6 carbon atoms.

2. A herbicide containing 1-(3-methoxymethyl-1,2-oxazol-5-yl)-ethyl-N-(3-chlorophenyl)-carbamate as active ingredient.

3. A herbicide containing 1-(3-methoxymethyl-1,2-oxazol-5-yl)-ethyl-N-(3-methylphenyl)-carbamate as active ingredient.

4. A herbicide containing 1-(3-methoxymethyl-1,2-oxazol-5-yl)-ethyl-N-(3-trifluoromethylphenyl)-carbamate as active ingredient.

5. A process for the manufacture of a 1,2-oxazolyl alkyl carbamate of the formula I as claimed in claim 1, wherein a 5-hydroxymethyl-1,2-oxazole of the formula II

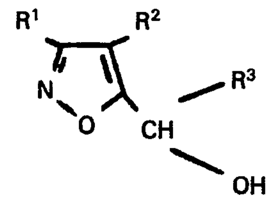

II

where $R^1$, $R^2$ and $R^3$ have the meanings given in claim 1, is reacted with an isocyanate of the formula III

$$OCN - R^4 \qquad III$$

where $R^4$ has the meanings given in claim 1, in the presence or absence of a diluent and at from —50° to + 150°C.

**Revendications pour les Etats contractants:** BE, CH, LI, DE, FR, GB, IT, LU, NL, SE

1. 1,2-oxazolylalkylcarbamates de formule I

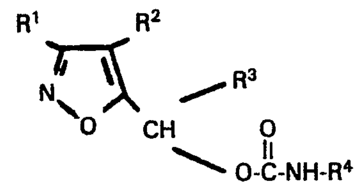

I

dans laquelle

$R^1$ représente un reste alcoxyalkyle ayant jusqu'à 3 atomes de carbone ou un reste alcoxycarbonyle ayant jusqu'à 5 atomes de carbone,

$R^2$ hydrogène ou un reste alkyle ayant jusqu'à 3 atomes de carbone, qui peut être substitué par un halogène,

$R^3$ hydrogène ou un reste alkyle ayant jusqu'à 3 atomes de carbone et

$R^4$ un reste phényle, qui peut être substitué par halogène, nitro, cyano ou des groupes alkyle, alcoxy, halogènoalkyle, halogène-alcoxy, alcanoyle ou alcoxy carbonyle, ayant jusqu'à 5 atomes de carbone, ou des groupes cycloalkyle ayant jusqu'à 6 atomes de carbone.

2. 1-(3-méthoxyméthyl-1,2-oxazolyl-5)-éthyl-N-(3-chlorophényl)-carbamate.

3. 1-(3-méthoxyméthyl-1,2-oxazolyl-5)-éthyl-N-(3-méthylphényl)-carbamate.

4. 1-(3-méthoxyméthyl-1,2-oxazolyl-5)-éthyl-N-(3-trifluorométhylphényl)-carbamate.

5. Procédé de préparation des 1,2-oxazolylalkylcarbamates de formule I selon la revendication 1, caractérisé par le fait qu'on fait réagir des 5-hydroxyméthyl-1,2-oxazols de formule II

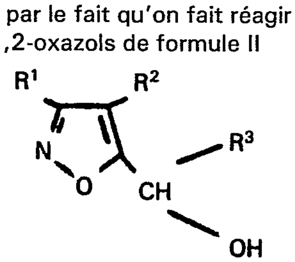

II

dans laquelle $R^1$, $R^2$ et $R^3$ ont les significations indiquées dans la revendication 1, avec des isocyanates de formule III

$$OCN - R^4 \qquad III$$

dans laquelle $R^4$ a les significations indiquées dans la revendication 1, éventuellement en présence d'un diluant, à une temperature comprise entre —50 et +150°C.

6. Herbicide, contenant au moins un 1,2-oxazolylalkylcarbamate de formule I selon la revendication 1.

7. Utilisation d'un 1,2-oxazolylalkylcarbamate de formule I selon la revendication 1, comme herbicide.

8. Procédé de lutte contre la croissance des plantes indésirables, caractérisé par le fait qu'on utilise un herbicide contenant un 1,2-oxazolylalkylcarbamate de formule I selon la revendication 1.

**Revendications pour l'Etat contractant:** AT

1. Herbicide contenant comme principe actif un 1,2-oxazolylalkylcarbamate de formule I

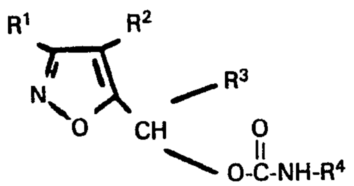

I

dans laquelle

$R^1$ représente un reste alcoxyalkyle ayant jusqu'à 3 atomes de carbone ou un reste alcoxycarbonyle ayant jusqu'à 5 atomes de carbone,

$R^2$ hydrogène ou un reste alkyle ayant jusqu'à 3 atomes de carbone, qui peut être substitué par un halogène,

$R^3$ hydrogène ou un reste alkyle ayant jusqu'à 3 atomes de carbone et

$R^4$ un reste phényle, qui peut être substitué par halogène, nitro, cyano ou des groupes alkyle, alcoxy, halogènoalkyle, halogène-alcoxy, alcanoyle ou alcoxy carbonyle, ayant jusqu'à 5 atomes de carbone, ou des groupes cycloalkyle ayant jusqu'à 6 atomes de carbone.

2. Herbicide contenant comme principe actif du 1-(3-méthoxyméthyl-1,2-oxazolyl-5)-éthyl-N-(3--chlorophényl)-carbamate.

3. Herbicide contenant comme principe actif du 1-(3-méthoxyméthyl-1,2-oxazolyl-5)-éthyl-N-(3-mé-thylphényl)-carbamate.

4. Herbicide contenant comme principe actif du 1-(3-méthoxyméthyl-1,2-oxazolyl-5)-éthyl-N-(3-trifluorométhylphényl)-carbamate.

5. Procédé de préparation des 1,2-oxazolylalkylcarbamates de formule I selon la revendication 1, caractérisé par le fait qu'on fait réagir des 5-hydroxyméthyl-1,2-oxazols de formule II

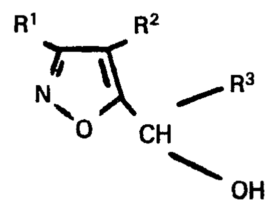

II

dans laquelle $R^1$, $R^2$ et $R^3$ ont les significations indiquées dans la revendication 1, avec des isocyanates de formule III

$$OCN - R^4 \qquad III$$

dans laquelle $R^4$ a les significations indiquées dans la revendication 1, éventuellement en présence d'un diluant, à une température comprise entre —50 et +150°C.